# EUROPEAN PATENT SPECIFICATION

(11) **EP 1 342 479 B1**
(45) Date of publication and mention of the grant of the patent: **15.12.2004**
(21) Application number: 03005118.9
(22) Date of filing: 07.03.2003
(51) Int. Cl.: A61M 1/16, A61M 1/34

(54) **Assembly for controlling and regulating flow of a dialysis solution in a haemodiafiltration process**
Einrichtung zur Kontrolle und Regulierung des Dialysatflusses in einem Hämodiafiltrationsverfahren
Dispositif de commande et de régulation du débit d'une solution de dialyse au cours d'un procédé d'hémodiafiltration

(30) Priority: 08.03.2002 IT BO20020119
(43) Date of publication of application: 10.09.2003
(73) Proprietor: BELLCO S.p.A., 20121 Milano (IT)
(72) Inventor: Cianciavicchia, Domenico, 64040 Cavuccio (IT); Fiorenzi, Andrea, 41013 Castelfranco Emilia (IT); Pradelli, Alessandro, 41034 Finale Emilia (IT)
(74) Representative: Cerbaro, Elena, Dr.

(56) References cited:
- EP-A- 0 516 152
- EP-A- 0 966 980
- WO-A-00/06217
- US-A- 5 660 722

## Description

The present invention relates to an assembly for controlling and regulating flow of a dialysis solution in a haemodiafiltration process.

Dialysis is a blood purifying process whereby toxic substances accumulated in the organism as a result of kidney failure are eliminated by transfer to a fluid with an electrolytic content similar to that of normal plasma containing no toxic substances, and which here and hereinafter is referred to as a "dialysis solution".

In conventional haemodialysis, the blood and dialysis solution are brought into contact in a filtering member comprising a semipermeable membrane. More specifically, the blood is circulated on one side of the membrane, and the dialysis solution is circulated in the opposite flow direction on the other side. That is, when drawn from the patient's arm, the blood flows along the so-called artery line into the dialysis filter, in which the dialysis solution circulates in the opposite direction and, by diffusion through the membrane, withdraws surplus toxic substances from the blood; and the purified blood issuing from the filter is restored to the patient along the so-called vein line.

In the haemodiafiltration process to which the invention refers, in addition to diffusion, by balancing the concentrations of two solutions brought into contact as described above, the blood is also purified by convection. Convection occurs whenever a pressure gradient is produced between the dialysis solution compartment and the blood compartment and in favour of the latter, so that plasma water flows through the semipermeable membrane, taking with it the toxic substances dissolved in it. For convection to be effective in terms of blood purification, a large amount of plasma water must be filtered, which means reinfusing a salt solution into the blood to maintain a suitable water balance. By the end of dialysis, however, a certain amount of fluid that the kidneys are no longer capable of eliminating in urine has been withdrawn from the patient, and which, known as "weight loss", must be taken into account when reinfusing the salt solution into the blood.

In haemodiafiltration, the dialysis solution circuit is normally regulated by a first and second pumping system upstream and downstream from the filter respectively, and reinfusion is performed along the vein line using a third pumping system which reinfuses a solution similar to the dialysis solution into the blood to restore the blood to its original volume minus the required weight loss, (see for example EP-A-0 516 152).

A major drawback of machines with the above equipment lies in failure of the first and second pumping systems to provide for both accurately regulating the dialysis solution, and accurate reinfusion calculated also as a function of the weight loss. The need for a third pumping system also has obvious drawbacks in terms of maintenance, size, and the limited extent to which the equipment can be used for more complex treatment.

It is an object of the present invention to provide a dialysis machine designed to calculate, to an adequate degree of precision, the amount of dialysis solution reinfused into the blood, and at the same to reduce the size of the machine itself.

According to the present invention, there is provided an assembly for controlling and regulating flow of a dialysis solution in a haemodiafiltration process, comprising at least a first pump and a second pump located respectively along a dialysis solution inlet branch and outlet branch of a haemodialysis filter; said assembly comprising differential reading means for reading the difference in flow of the dialysis solution in and out of said filter; and a central control unit for receiving information from said differential reading means. Said control unit is adapted to make a calculated variation in the delivery of one of said pumps, so as to periodically regulate the pressure of the dialysis solution in said filter, and impose alternate calculated flow, through a membrane of the filter, of both the dialysis solution and the plasma water in the blood for purification.

In a preferred embodiment of the assembly according to the present invention, the central control unit acts on a pump located along the dialysis solution outlet branch of the filter.

The assembly according to the present invention and as defined above provides for accurately reading and regulating the pressure of the dialysis solution in the haemodialysis filter, and so causing accurate, calculated convection of both the plasma water towards the dialysis solution compartment, and of the dialysis solution towards the blood compartment. The amount of reinfusion into the blood is therefore calculated accurately by working on the pressure of the dialysis solution in the filter, while at the same time releasing one pump for use in other types of dialysis treatment.

A non-limiting embodiment of the present invention will be described by way of example with reference to the accompanying drawings, in which:
Figure 1 shows a schematic diagram of a preferred embodiment of the assembly for controlling and regulating flow of a dialysis solution in a haemodiafiltration process;
Figure 2 shows a schematic diagram of the Figure 1 assembly applied to a combined haemodiafiltration-haemolipodialysis process.

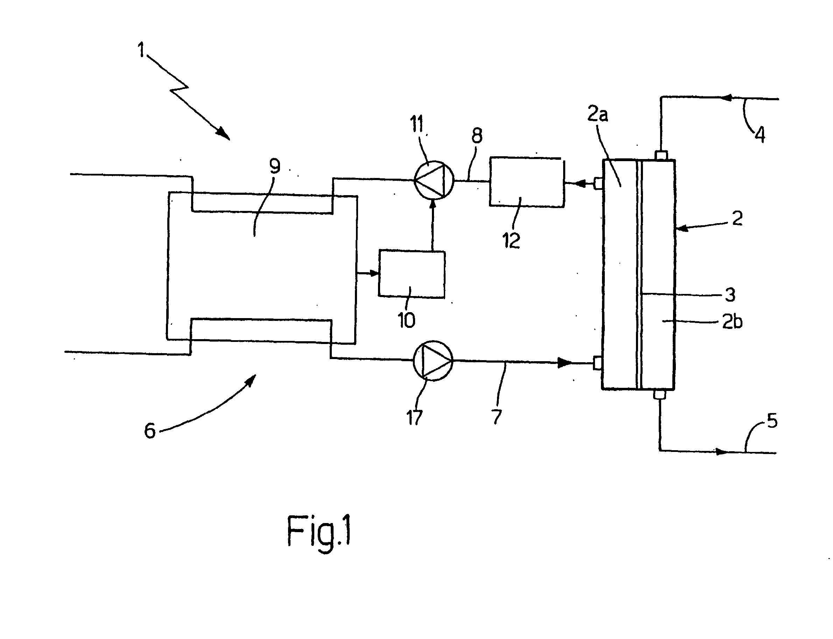

Number 1 in Figure 1 indicates as a whole a known dialysis machine (shown only partly), of which are shown schematically a haemodialysis filter 2 comprising a semipermeable membrane 3 dividing filter 2 into a dialysis solution compartment 2a and a blood compartment 2b; an artery line 4 for feeding blood into filter 2; a vein line 5 for feeding blood from filter 2 to the patient; and the assembly 6 for controlling and regulating dialysis solution flow according to the present invention.

Assembly 6 comprises an inlet branch 7 and an outlet branch 8 for feeding the dialysis solution to and from filter 2 respectively; a differential flowmeter 9 employing the Coriolis principle (known and therefore not described in detail) and involving portions of inlet branch 7 and outlet branch 8; and a central control unit 10 for receiving information from differential flowmeter 9 and accordingly operating (in known manner) a pumping system 11 acting on outlet branch 8. By maintaining a fixed delivery of a second pumping system 17 controlling dialysis solution flow to compartment 2a of filter 2, and by regulating outflow by regulating the delivery of pumping system 11, it is possible to regulate the pressure of the dialysis solution in compartment 2a of filter 2, and so regulate the amount and direction of fluid flowing by convection through membrane 3.

In actual use, central control unit 10 operates pumping system 11 to produce, inside filter 2, a calculated, periodic gradient inversion of the pressure in compartment 2a and the pressure in compartment 2b, and so produce a calculated, periodic flow of dialysis solution or plasma water through membrane 3. Obviously, in determining the pressure gradient causing the plasma water to flow from the blood to the dialysis solution in the blood, the weight loss determined by the specific requirements of the patient must be taken into account.

Assembly 6 also comprises a detector 12 located along dialysis solution outlet branch 8 to detect any damage to semipermeable membrane 3, by detecting the presence or absence of traces of haemoglobin along dialysis solution outlet branch 8 of filter 2. Since dialysis is compromised by any damage to semipermeable membrane 3, the membrane must be constantly monitored, and the entire filter 2 replaced if necessary.

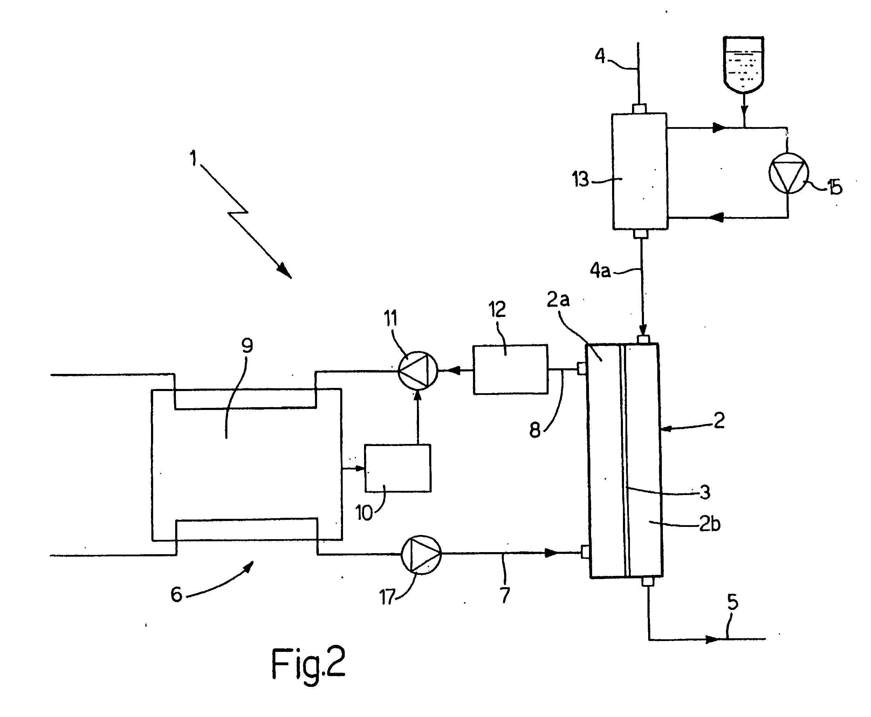

Figure 2 shows an assembly in accordance with the present invention applied to a combined haemodiafiltration-haemolipodialysis system, and any parts of which similar to those described with reference to Figure 1 are indicated using the same numbering system with no further description.

Artery line 4 is fitted with a haemolipodialysis filter 13, which produces purified blood using liposomes, which are brought into contact with the blood via a membrane in filter 13 as described in the Applicant's Patent Application EP 0966980.

Combining haemodiafiltration and haemolipodialysis enhances the effectiveness of dialysis as a whole. The combination described herein employs a peristaltic pump 15, which may advantageously be the one forming part of the known reinfusion system and made redundant by replacing the system with the assembly according to the present invention.

As will be obvious from the foregoing description, the assembly according to the present invention ensures reinfusion of a precise quantity of dialysis solution into the blood, while at the same time eliminating a pump and the dialysis solution circuit of which the pump forms part. Moreover, as shown in the combined haemodiafiltration-haemolipodialysis process in Figure 2, the pump made redundant by the assembly according to the invention may be put to other uses to enhance the efficiency of the dialysis operation as a whole.

Differential flowmeter 9 provides for determining the difference in flow in and out of filter 2 and vice versa, i.e. for determining extremely accurately the amount of fluid flowing from compartment 2a to compartment 2b, and vice versa.

Clearly, changes may be made to the assembly for controlling and regulating flow of a dialysis solution in a haemodiafiltration process as described herein without, however, departing from the scope of the accompanying Claims.

## Claims

1. An assembly (6) for controlling and regulating flow of a dialysis solution in a haemodiafiltration process, comprising at least a first pump (17) and a second pump (11) located respectively along a dialysis solution inlet branch (7) and outlet branch (8) of a haemodialysis filter (2); said assembly comprising differential reading means (9) for reading the difference in flow of the dialysis solution in and out of said filter (2); and a central control unit (10) for receiving information from said differential reading means (9), **characterized in that** said control unit is adapted to make a calculated variation in the delivery of one of said pumps (11, 17), so as to periodically regulate the pressure of the dialysis solution in said filter (2), and impose alternate calculated flow, through a membrane (3) of the filter (2), of both the dialysis solution and the plasma water in the blood for purification.

2. An assembly as claimed in Claim 1, **characterized in that** said pump (11) is located along said dialysis solution outlet branch (8) of the haemodialysis filter (2).

3. An assembly as claimed in Claim 1 and/or 2, **characterized in that** said differential reading means (9) read both the quantity of plasma water withdrawn from the blood, and the quantity of dialysis solution fed into the blood.

4. An assembly as claimed in any one of the foregoing Claims, **characterized in that** said differential reading means (9) are defined by a differential flowmeter (9)

5. An assembly as claimed in Claim 4, **characterized in that** said differential flowmeter (9) works on the Coriolis principle.

6. An assembly as claimed in any one of the foregoing Claims, **characterized by** comprising a detector (12) located along the dialysis solution outlet branch (8) to detect the presence of haemoglobin along the dialysis solution outlet branch (8).

7. An assembly as claimed in any one of the foregoing Claims, **characterized by** comprising a haemolipodialysis filter (13) upstream from said haemodialysis filter (2).

## Patentansprüche

1. Anordnung (6) zur Steuerung und Regulierung eines Flusses einer Dialyselösung in einem Hämodiafiltrations-Prozess, die wenigstens eine erste Pumpe (17) und eine zweite Pumpe (11) umfasst, die entlang eines Dialyselösungs-Zuflusszweiges (7) bzw. eines Dialyselösungs-Abflusszweiges (8) eines Hämodialysefilters (2) angeordnet sind, wobei die Anordnung Differenziallesemittel (9) zum Lesen der Strömungsdifferenz der Dialyselösung in den und aus dem Filter (2) umfasst, und eine zentrale Steuereinheit (10) zum Empfangen von Informationen von den Differenziallesemitteln (9) umfasst, **dadurch gekennzeichnet, dass** die zentrale Steuereinheit dazu geeignet ist, eine berechnete Veränderung der Fördermenge einer der Pumpen (11, 17) durchzuführen, um den Druck der Dialyselösung in dem Filter (2) periodisch zu regulieren und um einen alternierenden berechneten Fluss sowohl der Dialyselösung als auch des Plasmawassers des zu reinigenden Blutes durch eine Membran (3) des Filters (2) zu bewirken.

2. Anordnung nach Anspruch 1, **dadurch gekennzeichnet, dass** die Pumpe (11) entlang des Dialyselösungs-Ausflusszweiges (8) des Hämodialysefilters (2) angeordnet ist.

3. Anordnung nach Anspruch 1 und/oder 2, **dadurch gekennzeichnet, dass** die Differenziallesemittel (9) sowohl die dem Blut entzogene Menge an Plasmawasser als auch die dem Blut zugeführte Menge an Dialyselösung lesen.

4. Anordnung nach einem der vorhergehenden Ansprüche, **dadurch gekennzeichnet, dass** das die Differenziallesemittel (9) von einem Differenzialdurchflussmesser (9) gebildet sind.

5. Anordnung nach Anspruch 4, **dadurch gekennzeichnet, dass** der Differenzialdurchflussmesser (9) nach dem Coriolis-Prinzip arbeitet.

6. Anordnung nach einem der vorhergehenden Ansprüche, **dadurch gekennzeichnet, dass** sie einen Detektor (12) umfasst, der entlang des Dialyselösungs-Ausflusszweiges (8) angeordnet ist, um das Vorhandensein von Hämoglobin entlang des Dialyselösungs-Ausflusszweiges (8) zu erfassen.

7. Anordnung nach einem der vorhergehenden Ansprüche, **dadurch gekennzeichnet, dass** sie einen Hämolipodialysefilter (13) umfasst, der in Strömungsrichtung oberhalb des Hämodialysefilters (2) angeordnet ist.

## Revendications

1. Ensemble (6) pour commander et réguler l'écoulement d'une solution de dialyse dans un processus d'hémodiafiltration, comportant au moins une première pompe (17) et une seconde pompe (11) positionnées respectivement le long d'une branche d'entrée de solution de dialyse (7) et d'une branche de sortie (8) d'un filtre d'hémodialyse (2) ; ledit ensemble comportant des moyens de lecture différentielle (9), pour lire la différence d'écoulement de la solution de dialyse à l'intérieur et à l'extérieur dudit filtre (2), et une unité de commande centrale (10) pour recevoir des informations desdits moyens de lecture différentielle (9), **caractérisé en ce que** ladite unité de commande est adaptée pour effectuer une variation calculée du débit d'une desdites pompes (11, 17), de manière à réguler périodiquement la pression de la solution de dialyse dans ledit filtre (2), et imposer un écoulement calculé alterné, à travers une membrane (3) du filtre (2), à la fois de la solution de dialyse et de l'eau de plasma dans le sang pour une purification.

2. Ensemble selon la revendication 1, **caractérisé en ce que** ladite pompe (11) est positionnée le long de ladite branche de sortie de solution de dialyse (8) du filtre d'hémodialyse (2).

3. Ensemble selon la revendication 1 et/ou 2, **caractérisé en ce que** lesdits moyens de lecture différentielle (9) lisent à la fois la quantité d'eau de plasma extraite du sang, et la quantité de solution de dialyse délivrée dans le sang.

4. Ensemble selon l'une quelconque des revendications précédentes, **caractérisé en ce que** lesdits moyens de lecture différentielle (9) sont définis par un débitmètre différentiel (9).

5. Ensemble selon la revendication 4, **caractérisé en ce que** ledit débitmètre différentiel (9) fonctionne sur le principe de Coriolis.

6. Ensemble selon l'une quelconque des revendications précédentes, **caractérisé en ce qu'**il comporte un détecteur (12) positionné le long de la branche de sortie de solution de dialyse (8) pour détecter la présence d'hémoglobine le long de la branche de sortie de solution de dialyse (8).

7. Ensemble selon l'une quelconque des revendications précédentes, **caractérisé en ce qu'**il comporte un filtre d'hémolipodialyse (13) en amont dudit filtre d'hémodialyse (2).
